(19) 

Europäisches
Patentamt
European
Patent Office
Office européen
des brevets

(11) **EP 4 122 910 A1**

(12) **EUROPEAN PATENT APPLICATION**
published in accordance with Art. 153(4) EPC

(43) Date of publication:
**25.01.2023 Bulletin 2023/04**

(21) Application number: **21771622.4**

(22) Date of filing: **26.02.2021**

(51) International Patent Classification (IPC):
*C07C 51/00* (2006.01)   *B01D 61/02* (2006.01)
*B01D 61/14* (2006.01)   *B01J 31/24* (2006.01)
*B01J 31/40* (2006.01)   *B01J 38/00* (2006.01)
*C07C 53/06* (2006.01)

(52) Cooperative Patent Classification (CPC):
**B01D 61/02; B01D 61/14; B01J 31/24; B01J 31/40;
B01J 38/00; C07C 51/00; C07C 53/06;**
Y02P 20/584

(86) International application number:
**PCT/JP2021/007388**

(87) International publication number:
**WO 2021/187057 (23.09.2021 Gazette 2021/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2020  JP 2020046665**

(71) Applicant: **NITTO DENKO CORPORATION
Ibaraki-shi
Osaka 567-8680 (JP)**

(72) Inventors:
• **HIRANO Makoto
  Ibaraki-shi, Osaka 567-8680 (JP)**
• **IHARA Terukazu
  Ibaraki-shi, Osaka 567-8680 (JP)**
• **MATSUDA Hirokazu
  Ibaraki-shi, Osaka 567-8680 (JP)**

(74) Representative: **Hoffmann Eitle
Patent- und Rechtsanwälte PartmbB
Arabellastraße 30
81925 München (DE)**

(54) **FORMATE PRODUCTION METHOD AND FORMATE PRODUCTION SYSTEM**

(57)    The present invention pertains to a formate production method comprising: a first step for producing a formate by causing a reaction between hydrogen and carbon dioxide in a solution containing a solvent, a catalyst dissolved in the solvent, and a metal salt or an organic salt; and a second step for separating, by using a separation membrane, the catalyst from the reaction solution obtained from the first step. The catalyst includes at least one metal element selected from the group consisting of metal elements belonging to group 8, group 9, and group 10 of the periodic table.

EP 4 122 910 A1

**Description**

TECHNICAL FIELD

The present invention relates to a formate production method and a formate production system.

BACKGROUND ART

Due to problems such as global warming and fossil fuel depletion, hydrogen energy has been highly expected as next-generation energy.

Since a formic acid requires less energy for a dehydrogenation reaction and can be handled easily, the formic acid is considered to be an excellent compound as a hydrogen storage material and is attracting attention.

In order to use a formic acid as a hydrogen storage material, it is necessary to efficiently obtain a formic acid solution. Therefore, a method of producing a formic acid from carbon dioxide ($CO_2$) and hydrogen ($H_2$) in the presence of a catalyst has been studied.

In general, since a raw material of a catalyst is very expensive, it is desirable to separate and recover a catalyst from a reaction mixture and reuse the catalyst. When the used catalyst is a heterogeneous catalyst, separation is possible by a simple method such as filtration. However, when the used catalyst is a homogeneous catalyst, separation of the catalyst is difficult, and various separation methods have been studied.

For example, in Patent Literature 1, a method of separating and recovering an expensive catalyst from a reaction mixture using a homogeneous catalyst with high efficiency was studied. Patent Literature 1 describes a method of separating a homogeneous catalyst out of a reaction mixture using at least one membrane separation unit, in which, the reaction mixture containing the homogeneous catalyst and coming from a reaction zone is applied as a feed to the membrane separation unit, the homogeneous catalyst is depleted in a permeate of the membrane separation unit and enriched in a retentate of the membrane separation unit, and the retentate of the membrane separation unit is recirculated into the reaction zone, and describes a related device.

CITATION LIST

PATENT LITERATURE

Patent Literature 1: JP-T-2016-525925

SUMMARY OF INVENTION

TECHNICAL PROBLEM

Examples of the method of separating a formic acid and a catalyst from a reaction mixture include a method in which a formic acid is separated from a reaction solution by distillation, a method in which a catalyst is separated from a reaction solution by adsorption, and a method in which a formic acid solution and a catalyst solution are separated by layer separation.

However, when a formic acid is separated from a reaction solution by distillation, the catalyst is decomposed by heat. When a catalyst is separated from a reaction solution by adsorption, the catalytic activity decreases in a catalyst desorption step. The method in which a formic acid solution and a catalyst solution are separated by phase separation has a problem that the recovery efficiency of the catalyst is low.

The method described in Patent Literature 1 is a method of separating a homogeneous catalyst from a reaction solution in a hydroformylation reaction, and a method of separating, using a separation membrane, a catalyst from a reaction solution for producing a formate from carbon dioxide was not studied.

Therefore, the present invention provides a formate production method, by which a catalyst is separated by a separation membrane from a reaction solution in the production of the formate to recover the catalyst with high efficiency, a formate can be efficiently produced, and hydrogen can be stored in a state excellent in handling.

SOLUTION TO PROBLEM

As a result of intensive studies, the present inventors have found that the above problems can be solved, and have completed the present invention.

That is, the present invention is as follows.

[1] A formate production method, comprising:

a first step of producing a formate by causing a reaction between carbon dioxide and hydrogen in a solution containing a solvent, a catalyst dissolved in the solvent, and a metal salt or an organic salt; and
a second step of separating, by a separation membrane, the catalyst from a reaction solution obtained in the first step, wherein
the catalyst contains at least one metal element selected from the group consisting of metal elements belonging to Group 8, Group 9, and Group 10 of a periodic table.

[2] The formate production method according to [1], wherein a base concentration in the reaction solution in the second step is 0.1 to 0.8 mol/L.

[3] The formate production method according to [1] or [2], wherein a pH of the reaction solution in the second step is 6.3 to 7.3.

[4] The formate production method according to any one of [1] to [3], wherein a formate concentration with respect to a base concentration in the reaction solution in the second step is 0.5 or more.

[5] The formate production method according to any one of [1] to [4], wherein the catalyst separated in the second step is reused in the first step.

[6] The formate production method according to any one of [1] to [5], wherein the separation membrane includes a separation membrane unit including a reverse osmosis membrane.

[7] The formate production method according to any one of [1] to [6], wherein the metal element is Ru, Ir, Fe, Ni, or Co.

[8] The formate production method according to any one of [1] to [7], wherein the metal element is Ir or Ru.

[9] A formate production system that produces a formate, the system comprising:

a formate production device configured to produce a formate by causing a reaction between carbon dioxide and hydrogen in a solution containing a solvent, a catalyst dissolved in the solvent, and a metal salt or an organic salt; and
a separation device configured to separate the catalyst from a reaction solution obtained by the reaction, wherein
the catalyst contains at least one metal element selected from the group consisting of metal elements belonging to Group 8, Group 9, and Group 10 of a periodic table.

ADVANTAGEOUS EFFECTS OF INVENTION

According to the present invention, it is possible to provide a formate production method, by which a catalyst is separated by a separation membrane from a reaction solution in the production of the formate to recover the catalyst with high efficiency, a formate can be efficiently produced, and hydrogen can be stored in a state excellent in handling.

BRIEF DESCRIPTION OF DRAWINGS

[FIG. 1] FIG. 1 is a diagram showing an example of a formate production system according to an embodiment of the present invention.
[FIG. 2] FIG. 2 is a diagram showing an embodiment of the present invention.

DESCRIPTION OF EMBODIMENTS

Hereinafter, an embodiment of the present invention will be described in detail.
A formate production method according to an embodiment of the present invention includes a first step of producing a formate by causing a reaction between carbon dioxide and hydrogen in a solution containing a solvent, a catalyst dissolved in the solvent, and a metal salt or an organic salt; and a second step of separating, by a separation membrane, the catalyst from a reaction solution obtained in the first step. The catalyst contains at least one metal element selected from the group consisting of metal elements belonging to Group 8, Group 9, and Group 10 of a periodic table.
In the formate production method according to the embodiment of the present invention, the catalyst separated in the second step is preferably reused in the first step.
According to the formate production method and a formate production system according to the embodiment of the present invention, a catalyst can be recovered with high efficiency from a reaction solution in the production of a formate, a formate can be efficiently produced, and hydrogen can be stored in a state excellent in handling.

[First Step]

The first step is a step of producing a formic acid by causing a reaction between carbon dioxide and hydrogen in a solution containing a solvent, a catalyst dissolved in the solvent, and a metal salt or an organic salt. Since the catalyst and the metal salt or the organic salt are dissolved in the solvent, the catalyst effect is excellent, a formate can be generated with high efficiency, and hydrogen can be stored as a formate. The formate has a high hydrogen storage density and can be easily handled. When the formate is used as a hydrogen storage material, the formate is safe and stable as a chemical substance. Therefore, there is an advantage that the formate can be stored for a long period of time. A formate aqueous solution produced in this step can be subjected to the second step.

(Metal Salt or Organic Salt)

Examples of the metal salt according to the embodiment of the present invention include an alkali metal salt and an alkaline earth metal salt, and an alkali metal salt is preferable.

As the alkali metal salt, an inorganic salt of an alkali metal can be used. The alkali metal salt may be used alone or in combination of two or more thereof.

Examples of alkali metal ions constituting a cation moiety of the alkali metal salt include ions of lithium, sodium, potassium, rubidium, and cesium. Among these alkali metal ions, a sodium ion or a potassium ion is preferable.

An anion moiety of the alkali metal salt is not particularly limited as long as it can produce an alkali metal formate. Examples of the anion moiety include a hydroxide ion ($OH^-$), a chloride ion ($Cl^-$), a bromide ion ($Br^-$), an iodide ion ($I^-$), a nitrate ion ($NO_3^-$), a sulfate ion ($SO_4^{2-}$), a phosphate ion ($PO_4^{2-}$), a borate ion ($BO_3^{3-}$), a hydrogen carbonate ion ($HCO_3^-$), and a carbonate ion ($CO_3^{2-}$). It is preferable to include at least one selected from these.

Specific examples of the alkali metal salt include lithium hydroxide, sodium hydroxide, potassium hydroxide, rubidium hydroxide, cesium hydroxide, lithium chloride, sodium chloride, potassium chloride, rubidium chloride, cesium chloride, lithium sulfate, sodium sulfate, potassium sulfate, rubidium sulfate, cesium sulfate, lithium hydrogen carbonate, sodium hydrogen carbonate, potassium hydrogen carbonate, rubidium hydrogen carbonate, cesium hydrogen carbonate, lithium carbonate, sodium carbonate, potassium carbonate, rubidium carbonate, and cesium carbonate. From the viewpoint that by-products are not easily mixed when the alkali metal formate is produced, and an operation after the second step is not complicated, alkali metal hydroxide, alkali metal hydrogen carbonate, and alkali metal carbonate are preferable, sodium hydroxide, potassium hydroxide, sodium hydrogen carbonate, potassium hydrogen carbonate, sodium carbonate, and potassium carbonate are more preferable, and sodium hydrogen carbonate and potassium hydrogen carbonate are particularly preferable.

Examples of the organic salt include diazabicycloundecene and triethylamine.

The content of the metal salt or the organic salt used in the formate production step is preferably 0.05 mol/L or more, more preferably 0.1 mol/L or more, and still more preferably 0.2 mol/L or more, from the viewpoint of increasing the production amount of the formate. From the viewpoint of resource saving, the content of the metal salt or the organic salt is preferably 20 mol/L or less, more preferably 15 mol/L or less, and still more preferably 10 mol/L or less.

The method for producing a formate in an aqueous solution using carbon dioxide in the presence of a metal salt or an organic salt is not particularly limited, and may be a method in which carbon dioxide is hydrogenated (allowed to react with hydrogen) in the presence of a metal salt or an organic salt, a method in which carbon dioxide is electrolyzed in the presence of a metal salt or an organic salt, a method in which carbon dioxide is reduced by a photocatalyst in the presence of a metal salt or an organic salt, a method in which carbon dioxide is reduced by a biological means such as an enzyme in the presence of a metal salt or an organic salt, or a method in which each method is performed in the absence of a metal salt or an organic salt to produce a formic acid, and then the formic acid is allowed to react with the metal salt or the organic salt to produce a formate.

(Catalyst)

The catalyst used in the embodiment of the present invention contains at least one metal element selected from the group consisting of metal elements belonging to Group 8, Group 9, and Group 10 of a periodic table (hereinafter, may be simply referred to as a metal element). Specific examples of the metal element include Fe, Ru, Os, Hs, Co, Ir, Mt, Ni, Pd, Pt, and Ds. From the viewpoint of catalytic performance, Ru, Ir, Fe, Ni, and Co are preferable, and Ru and Ir are more preferable.

Since the first step needs to be performed in a solution containing a catalyst dissolved in a solvent, the catalyst is preferably a homogeneous catalyst. The catalyst used in the embodiment of the present invention is preferably soluble in water, an organic solvent, or the like, and is more preferably a compound containing a metal element (metal element compound).

Examples of the metal element compound include a salt of a metal element with an inorganic acid such as a hydride salt, an oxide salt, a halide salt (such as a chloride salt), a hydroxide salt, a carbonate salt, a hydrogen carbonate salt, a sulfate salt, a nitrate salt, a phosphate salt, a borate salt, a harate salt, a perharate salt, a harite salt, a hypoharite salt, and a thiocyanate salt; a salt of a metal element with an organic acid such as an alkoxide salt, a carboxylate salt (such as an acetate salt and a (meth)acrylate salt), and a sulfonate salt (such as a trifluoromethanesulfonate salt); a salt of a metal element with an organic base such as an amide salt, a sulfonamide salt, and a sulfonimide salt (such as a bis(trifluoromethanesulfonyl)imide salt); a complex salt such as an acetylacetone salt, a hexafluoroacetylacetone salt, a porphyrin salt, a phthalocyanine salt, and a cyclopentadiene salt; complexes or salts containing one or more of a nitrogen compound containing a chain amine, a cyclic amine, an aromatic amine, and the like, a phosphorus compound, a compound containing phosphorus and nitrogen, a sulfur compound, carbon monoxide, carbon dioxide, water, and the like. These compounds may be either a hydrate or an anhydride, and are not particularly limited. Among these, a halide salt, a complex containing a phosphorus compound, a complex containing a nitrogen compound, and a complex or salt containing a compound containing phosphorus and nitrogen are preferable, from the viewpoint of further enhancing the production efficiency of a formate.

These may be used alone or in combination of two or more thereof.

As the metal element compound, a commercially available metal element compound can be used, or a metal element compound produced by a known method or the like can also be used.

As a known method, for example, methods described in JP-A-2018-114495, Yuichiro Himeda; Nobuko Onozawa-Komatsuzaki; Satoru Miyazawa; Hideki Sugihara; Takuji Hirose; Kazuyuki Kasuga. Chem. Eur. J. 2008, 14, 11076-11081, Wan-Hui Wang; Jonathan F. Hull; James T. Muckerman; Etsuko Fujita; Yuichiro Himeda. Energy Environ. Sci. 2012, 5, 7923-7926, Yuichiro Himeda; Nobuko Onozawa-Komatsuzaki; Hideki Sugihara; Kazuyuki Kasuga, Organometallics 2007, 26, 3, 702-712 can be used.

For example, the following homogeneous catalyst can be synthesized by the known method described above. [Cp*Ir(4,4-dihydroxy-2,2-bipyridine)(OH$_2$)]SO$_4$ : Ir-4DHBP[(C$_6$Me$_6$)Ru(4,4-dihydroxy-2,2-bipyridine)Cl]Cl Ru-4DHBP[Cp*Ir(6,6'-Dihydroxyl-2,2'-bipyridine)(OH$_2$)]SO$_4$ : Ir-6DHBP

The amount of the catalyst to be used is not particularly limited as long as a formic acid or a formate can be produced. When the metal element compound is used as the catalyst, the amount of the metal element compound to be used is preferably 0.1 $\mu$mol or more, more preferably 0.5 $\mu$mol or more, and still more preferably 1 $\mu$mol or more with respect to 1 L of the solvent in order to sufficiently express the catalytic function. From the viewpoint of cost, the amount of the metal element compound to be used is preferably 1 mol or less, more preferably 10 mmol or less, and still more preferably 1 mmol or less. When two or more of the metal element compounds are used, the total amount of the metal element compounds to be used may be within the above range.

(Solvent)

The solvent according to the embodiment of the present invention is not particularly limited as long as a catalyst, which is dissolved in the solvent, becomes uniform. Water, ethylene glycol, polyethylene glycol, glycerin, methanol, ethanol, propanol, pentanol, dimethyl sulfoxide, tetrahydrofuran, benzene, toluene, and the like can be used, but more preferably water, ethylene glycol, polyethylene glycol, glycerin can be used, and even more preferably water can be used. Further, after a formate is produced using a mixed solvent of water and a solvent miscible with water, the solvent miscible with water may be distilled off to form an aqueous solution of the formate.

(Carbon Dioxide and Hydrogen)

As the hydrogen used in the embodiment of the present invention, either a hydrogen gas cylinder or liquid hydrogen can be used. As a hydrogen supply source, for example, hydrogen generated in a smelting process of steel-making, hydrogen generated in a production process of Soda, or the like can be used. Hydrogen generated by electrolysis of water can also be used.

As the carbon dioxide used in the embodiment of the present invention, a carbon dioxide gas cylinder, liquid carbon dioxide, supercritical carbon dioxide, dry ice, and the like can be used.

A hydrogen gas and a carbon dioxide gas may be introduced into the reaction system individually or as a mixed gas.

It is preferable that the proportion of hydrogen and carbon dioxide to be used is the same on a molar basis or that hydrogen is excessive.

When a hydrogen cylinder is used as the hydrogen used in the formate production method according to the embodiment of the present invention, the pressure is preferably 0.1 MPa or more, more preferably 0.2 MPa or more, and still more preferably 0.5 MPa or more, from the viewpoint of sufficiently expressing reactivity. The pressure is preferably 50 MPa or less, more preferably 20 MPa or less, and still more preferably 10 MPa or less because the size of the equipment tends to be large.

The pressure of carbon dioxide used in the formate production method according to the embodiment of the present invention is preferably 0.1 MPa or more, more preferably 0.2 MPa or more, and still more preferably 0.5 MPa or more, from the viewpoint of sufficiently expressing reactivity. The pressure is preferably 50 MPa or less, more preferably 20 MPa or less, and still more preferably 10 MPa or less because the size of the equipment tends to be large.

(Reaction Conditions)

Reaction conditions in the formate production method according to the embodiment of the present invention are not particularly limited, and the reaction conditions can be appropriately changed during the reaction process. A form of a reaction vessel used for the reaction is not particularly limited.

A reaction temperature is not particularly limited, but is preferably 30°C or higher, more preferably 40°C or higher, and still more preferably 50°C or higher, in order to allow the reaction to proceed efficiently. From the viewpoint of energy efficiency, the temperature is preferably 200°C or lower, more preferably 150°C or lower, and still more preferably 100°C or lower.

A reaction time is not particularly limited, but is, for example, preferably 0.5 hours or more, more preferably 1 hour or more, and still more preferably 2 hours or more from the viewpoint of obtaining the enough production amount of the formate. From the viewpoint of cost, the reaction time is preferably 24 hours or less, more preferably 12 hours or less, and still more preferably 6 hours or less.

A method of introducing carbon dioxide, hydrogen, a catalyst, a solvent, and the like used for the reaction into the reaction vessel is not particularly limited. All the raw materials and the like may be introduced collectively, some or all the raw materials and the like may be introduced stepwise, or some or all the raw materials and the like may be introduced continuously. An introduction method in which all these methods are combined may be used.

[Second Step]

The second step according to the embodiment of the present invention is a step of separating, by a separation membrane, a catalyst from a reaction solution obtained in the first step.

The catalyst is separated from the reaction solution as a catalyst solution by a separation membrane (membrane separation). The catalyst separated from the reaction solution by the separation membrane may be reused.

Since membrane separation can be performed under mild conditions, the membrane separation is suitable for separation of a homogeneous catalyst having low thermal stability or a compound difficult to be separated by distillation. A catalyst typically has low durability, the catalytic activity is likely to decrease during post-treatment such as purification after the production of a formate, and it is difficult to reuse the catalyst. However, in the embodiment of the present invention, since the catalyst is separated by the separation membrane in the second step, a decrease in the catalytic activity can be prevented, only the catalyst can be separated from the reaction mixture at a high yield, and the catalyst can be reused.

When the catalyst is separated in the second step, it is preferable that the catalyst is dissolved in water, an organic solvent or the like, and it is more preferable that the water, the organic solvent or the like contains a catalyst metal.

The proportion of the catalyst separated in the second step used in a solution in the first step is not particularly limited. A part of a total catalyst may be the catalyst separated in the second step, or all of the total catalyst may be the catalyst separated in the second step. The proportion of the catalyst separated in the second step to the total catalyst is preferably 50 mass% or more, more preferably 70 mass% or more, and should be as much as possible from the viewpoint of catalyst cost.

The separation membrane used in the second step is not particularly limited as long as the catalyst can be separated from the reaction solution. It is preferable to include a separation membrane unit.

The separation membrane unit may have a separation membrane housed in a housing. Examples of a form of the separation membrane unit include a flat membrane plate frame type, a pleated type, and a spiral type.

The separation membrane is not particularly limited as long as it allows a formate aqueous solution to permeate and hardly allows metal ions contained in the catalyst to permeate. The separation membrane may be a reverse osmosis membrane (RO membrane), a nano filtration membrane (NF membrane), a micro filtration membrane (MF membrane), or an ultra filtration membrane (UF membrane), but an RO membrane or an NF membrane is preferably used, from the viewpoint of the size of a pore diameter.

The pore diameter of the separation membrane is preferably 1 Å or more, more preferably 2 Å or more, and still more preferably 5 Å or more, from the viewpoint of a permeation rate of the aqueous solution. From the viewpoint of a catalyst recovery rate, the pore diameter is preferably 50 Å or less, more preferably 20 Å or less, and still more preferably 10 Å or less.

Commercially available products can be used as the separation membrane. Examples thereof include NANO-SW manufactured by Nitto Denko Corporation, PRO-XS 1 manufactured by Nitto Denko Corporation, ESPA-DSF manufactured by Nitto Denko Corporation, CPA7 manufactured by Nitto Denko Corporation, and SWC5-LD manufactured by Nitto Denko Corporation. NANO-SW manufactured by Nitto Denko Corporation is preferably used.

The base concentration in the reaction solution in the second step is preferably 0.1 mol/L or more, more preferably 0.3 mol/L or more, and still more preferably 0.5 mol/L or more. The base concentration is preferably 1 mol/L or less, more preferably 0.8 mol/L or less, and still more preferably 0.5 mol/L or less. When the base concentration is 0. 1 to 1 mol/L, the recovery rate of the catalyst can be improved. The reason why the recovery rate of the catalyst is improved by setting the base concentration to the above range is not clear, but the inventors presume that the permeability of the catalyst to the membrane is changed due to a change of a degree of ionization of the catalyst caused by the difference in the base concentration.

The formate concentration with respect to the base concentration in the reaction solution in the second step is preferably 0.2 or more, and more preferably 0.5 or more. The reason why the recovery rate of the catalyst is improved by the increase of the formate concentration in the reaction solution is considered to be that the permeability of the catalyst to the membrane is changed due to a change of an ionization state of the catalyst caused by the difference in the base concentration. Therefore, the formate concentration with respect to the base concentration is more preferably 0.5 or more. The upper limit of the formate concentration with respect to the base concentration is not particularly limited.

The pH of the reaction solution in the reaction solution in the second step is preferably 6.3 or more in order to prevent deterioration of the separation membrane due to acidic conditions. The pH of the reaction solution is preferably 8 or less, more preferably 7.7 or less, and still more preferably 7.3 or less, in order to prevent deterioration of the separation membrane due to basic conditions.

The second step can be performed, for example, using a separation device equipped with a pressure-resistant vessel under normal pressure or pressure. The pressure in the second step can be adjusted by introducing an inert gas such as nitrogen gas into the pressure-resistant vessel from a cylinder connected to the pressure-resistant vessel.

The pressure in the second step is more preferably 0.1 MPa or more, and still more preferably 0.3 MPa or more, from the viewpoint of the permeation rate of the solution. From the viewpoint of energy cost due to the membrane separation, the pressure is preferably 10 MPa or less, more preferably 8 MPa or less, and still more preferably 6 MPa or less.

[Formic Acid Production System]

The formate production system according to the embodiment of the present invention is a formate production system for producing a formate. The formate production system includes: a formate production device configured to produce a formate by causing a reaction between carbon dioxide and hydrogen in a solution containing a solvent, a catalyst dissolved in the solvent, and a metal salt or an organic salt; and a separation device configured to separate the catalyst from a reaction solution obtained by the reaction. The catalyst contains at least one metal element selected from the group consisting of metal elements belonging to Group 8, Group 9, and Group 10 of a periodic table.

The formate production system according to the embodiment of the present invention may further include a flow path through which a solution in the formate production device is supplied from the formate production device to a separation device, and a flow path through which a catalyst separated by the separation device is supplied to the formate production device.

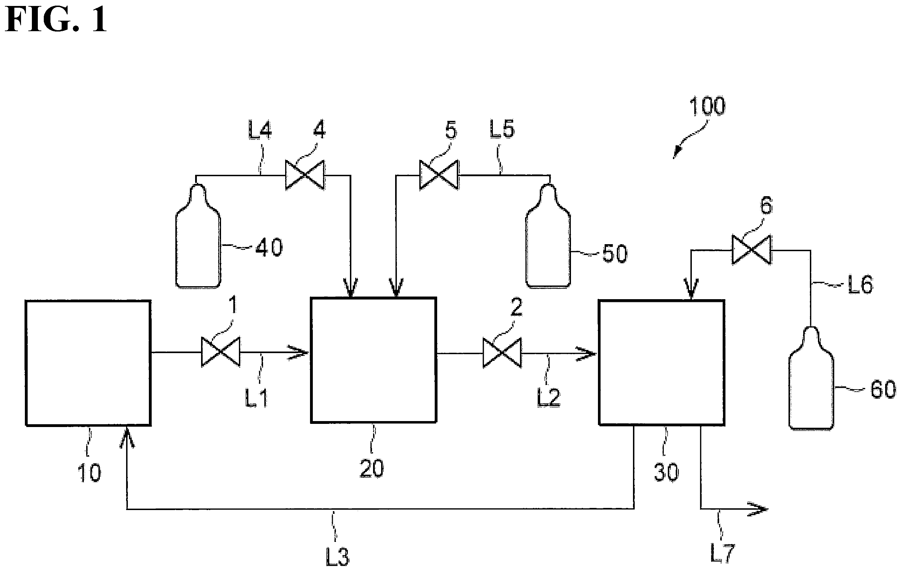

FIG. 1 is a diagram showing an example of the formate production system according to the embodiment of the present invention.

A formate production system 100 shown in FIG. 1 includes a formate production device 20 and a separation device 30, and may further include a solution preparation device 10, in which a catalyst is dissolved in a solvent and a metal salt or an organic salt is mixed thereto, a carbon dioxide cylinder 40 that supplies carbon dioxide to the formate production device 20, a hydrogen cylinder 50 that supplies a hydrogen gas, and a nitrogen cylinder 60 that adjusts a pressure in the separation device 30. The pressure can be adjusted by a valve 6 provided in a flow path L6.

The formate production system 100 may include a flow path L1, through which the solution containing a solvent, a catalyst dissolved in the solvent, and a metal salt or an organic salt is supplied from the solution preparation device 10 to the formate production device 20, and a flow path L2, through which the reaction solution is supplied from the formate production device 20 to the separation device 30. The formate production system 100 may include a flow path L4, through which carbon dioxide is supplied from the carbon dioxide cylinder 40 to the formate production device 20 and a flow path L5, through which hydrogen is supplied from the hydrogen cylinder 50 to the formate production device 20.

The formate production system 100 may further include a flow path L3, through which the catalyst solution separated by the separation device 30 is supplied to the solution preparation device 10 and a flow path L7, through which a permeated liquid separated by the separation device 30 is recovered.

The flow path L1 may be provided with a valve 1. The valve 1 can adjust the amount of the solution extracted from the solution preparation device 10 and circulated to the formate production device 20.

The flow path L2 may be provided with a valve 2. The valve 2 can adjust the amount of the reaction solution to be supplied to the separation device 30.

The flow path L4 and the flow path L5 may be provided with a valve 4 and a valve 5, respectively. The flow path L4 and the flow path L5 can adjust the amounts of a carbon dioxide gas and a hydrogen gas supplied to the formate production device 20.

According to the formate production method and the formate production system of the present embodiment, since the solution in the reactor can be separated as a catalyst solution by a simple operation, an expensive catalyst can be reused, and the formate can be efficiently produced.

Hereinafter, the present invention will be described in detail with reference to Examples and Comparative Examples. However, the present invention is not limited to these Examples.

[Example]

Hereinafter, the present invention will be specifically described with reference to Examples, but the present invention is not limited to these Examples.

<Synthesis of Homogeneous Catalyst>

By using methods described in JP-A-2018-114495, Yuichiro Himeda; Nobuko Onozawa-Komatsuzaki; Satoru Miyazawa; Hideki Sugihara; Takuji Hirose; Kazuyuki Kasuga. Chem. Eur. J. 2008, 14, 11076-11081, Wan-Hui Wang; Jonathan F. Hull; James T. Muckerman; Etsuko Fujita; Yuichiro Himeda. Energy Environ. Sci. 2012, 5, 7923-7926, Yuichiro Himeda; Nobuko Onozawa-Komatsuzaki; Hideki Sugihara; Kazuyuki Kasuga, Organometallics 2007, 26, 3, 702-712, the following homogeneous catalyst was synthesized.
[Cp*Ir(4,4-dihydroxy-2,2-bipyridine)(OH$_2$)]SO$_4$ : Ir-4DHBP[(C$_6$Me$_6$)Ru(4,4-dihydroxy-2,2-bipyridine)Cl]Cl Ru-4DHBP[Cp*Ir(6,6'-Dihydroxyl-2,2'-bipyridine)(OH$_2$)]SO$_4$ : Ir-6DHBP

[Examples 1 to 11 and Comparative Examples 1 to 3]

<Preparation of Test Solution>

A formic acid, sodium hydrogen carbonate as a metal salt, and a catalyst were mixed so that a catalyst metal concentration, a base concentration, and a formic acid concentration were as shown in Table 1, and a test solution was prepared using ion exchange water. The pH of the prepared test solution was measured using LAQUA twin-pH-11 (Horiba Advanced Techno Co., Ltd.) (measurement accuracy pH ± 0.1).

<Separation by Separation Membrane>

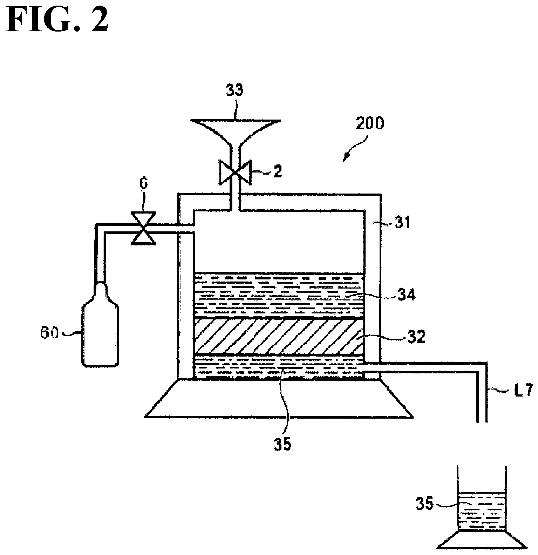

As shown in FIG. 2, a separation membrane 32 shown in Table 1 was installed as a separation membrane 32 in a lower portion of a pressure-resistant vessel 31 connected to the nitrogen cylinder 60 in a separation device 200. 60 mL of the test solution shown in Table 1 was introduced from a solution inlet 33 of the pressure-resistant vessel 31, and the valve 2 of the solution inlet 33 was closed. The valve 6 of the nitrogen cylinder 60 was opened, and a nitrogen gas was introduced until the pressure in the pressure-resistant vessel 31 reached about 4 MPa.

50 mL of a permeated liquid which passed through the separation membrane 32 was collected. The separation device 200 may include the flow path L7, through which a separated permeated liquid 35 was recovered.

About 10 mL of a solution (catalyst solution) 34 remaining in the vessel, which did not pass through the separation membrane, was recovered.

<Measurement of Catalyst Metal Concentration>

The catalyst metal concentration in the test solution and the catalyst metal concentration in the permeated liquid were measured by inductively coupled plasma-mass spectrometry (ICP-MS).

A catalyst loss rate was calculated by the following formula.

$$\text{Catalyst loss rate (\%)} = (\text{Cp} / \text{Cb}) \times 100$$

Cp: Catalyst metal concentration (ppm) in permeated liquid
Cb: Catalyst metal concentration (ppm) in test solution

Examples and comparative examples described above are shown in Table 1.

[Table 1]

Table 1

| | | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 |
|---|---|---|---|---|---|---|---|---|---|---|---|---|---|---|---|
| Solution conditions | Catalyst | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP | Ir-4DHBP |
| | Catalyst metal concentration in test solution (ppm) | 19 | 19 | 18 | 18 | 19 | 20 | 20 | 19 | 19 | 19 | 19 | 20 | 20 | 20 |
| | Base | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ | $NaHCO_3$ |
| | Base concentration (mol/L) | 0.3 | 0.3 | 0.3 | 0.5 | 0.5 | 0.5 | 0.7 | 0.7 | 1.0 | 1.0 | 1.0 | 0.1 | 0.5 | 1.0 |
| | Formic acid concentration (mol/L) | 0.06 | 0.15 | 0.24 | 0.10 | 0.25 | 0.40 | 0.14 | 0.56 | 0.20 | 0.50 | 0.80 | - | - | - |
| | Formic acid concentration / base concentration | 0.20 | 0.50 | 0.80 | 0.20 | 0.50 | 0.80 | 0.20 | 0.80 | 0.20 | 0.50 | 0.80 | - | - | - |
| | pH | 7.3 | 6.9 | 6.3 | 7.3 | 6.9 | 6.6 | 7.2 | 7.0 | 7.7 | 7.5 | 7.2 | 8.0 | 8.2 | 8.3 |
| Separation membrane | | NANO-SW | NANO-SW | NANO-SW | NANO-SW | NANO-SW | NANO-SW | NANO-SW | NANO-SW | NANO-SW | NANO-SW | NANO-SW | NANO-SW | NANO-SW | NANO-SW |
| Catalyst metal concentration in permeated liquid (ppm) | | 1.0 | 0.58 | 0.11 | 0.22 | 0.17 | 0.10 | 1.4 | 0.64 | 3.1 | 2.6 | 1.8 | 3.6 | 3.6 | 3.7 |
| Catalyst loss rate (%) | | 5.0 | 3.0 | 0.60 | 1.2 | 0.90 | 0.50 | 7.1 | 3.3 | 16 | 14 | 9.3 | 18 | 18 | 18 |

As shown in Table 1, it was confirmed that in Examples 1 to 11, in which the formate was contained in the solution to be separated, a catalyst loss rate was lower than that in Comparative Examples 1 to 3, in which the formate was not contained in the solution.

[Examples 12 to 18]

Separation was performed in the same manner as in Examples 1 to 11, except that a formic acid, sodium hydrogen carbonate or potassium hydrogen carbonate as a metal salt, and a catalyst were mixed so that a catalyst metal concentration, a base concentration, and a formic acid concentration were as shown in Table 2, and a test solution was prepared using ion exchange water. The results are shown in Table 2.

[Table 2]

Table 2

| | | Example 12 | Example 13 | Example 14 | Example 15 | Example 16 | Example 17 | Example 18 |
|---|---|---|---|---|---|---|---|---|
| Solution conditions | Catalyst | Ir-6DHBP | Ir-6DHBP | Ru-4DHBP | Ru-4DHBP | Ir-4DHBP | Ir-4DHBP | Ir-4DHBP |
| | Catalyst metal concentration in test solution (ppm) | 17 | 20 | 21 | 20 | 20 | 20 | 20 |
| | Base | $NaHCO_3$ | $NaHCO_3$ | $NaHCO_3$ | $NaHCO_3$ | $KHCO_3$ | $KHCO_3$ | $NaHCO_3$ |
| | Base concentration (mol/L) | 0.5 | 1.0 | 0.5 | 1.0 | 0.5 | 1.0 | 0.5 |
| | Formic acid concentration (mol/L) | 0.40 | 0.80 | 0.40 | 0.80 | 0.40 | 0.80 | 0.40 |
| | Formic acid concentration / base concentration | 0.80 | 0.80 | 0.80 | 0.80 | 0.80 | 0.80 | 0.80 |
| | pH | 6.6 | 7.2 | 6.7 | 7.2 | 6.6 | 7.1 | 6.7 |
| Separation membrane | | NANO-SW | NANO-SW | NANO-SW | NANO-SW | NANO-SW | NANO-SW | PRO-XS1 |
| Catalyst metal concentration in permeated liquid (ppm) | | 0.01 | 0.02 | 0.15 | 1.6 | 0.26 | 2.6 | 0.16 |
| Catalyst loss rate (%) | | 0.06 | 0.10 | 0.70 | 8.2 | 1.3 | 13 | 0.80 |

As shown in Table 2, it was confirmed that even in Examples 12 and 13, in which the catalyst was Ir-6DHBP and Examples 14 and 15, in which the catalyst was Ru-4DHBP, a low catalyst loss rate could be obtained in the same manner. It was confirmed that in Examples 16 and 17, in which potassium hydrogen carbonate was used as a metal salt, and in Example 18, in which a separation membrane (Nano-SW: manufactured by Nitto Denko Corporation, PRO-XS1: manufactured by Nitto Denko Corporation) different from the other examples was used, a low catalyst loss rate could be obtained in the same manner.

INDUSTRIAL APPLICABILITY

According to the present invention, it is possible to provide a formate production method, by which a catalyst is separated by a separation membrane from a reaction solution in the production of the formate to recover the catalyst with high efficiency, a formate can be efficiently produced, and hydrogen can be stored in a state excellent in handling.
Although the present invention has been described in detail using specific embodiments, it will be apparent to those skilled in the art that various modifications and variations are possible without departing from the spirit and scope of the present invention.
The present application is based on the Japanese patent application (Japanese Patent Application No. 2020-046665) filed on March 17, 2020, and the contents thereof are incorporated herein as reference.

REFERENCE SIGNS LIST

1, 2, 4, 5, 6 Valve
10 Solution preparation device
20 Formate production device
30, 200 Separation device
31 Pressure-resistant vessel
32 Separation membrane
33 Solution inlet
34 Solution
35 Permeated liquid
40 Carbon dioxide cylinder
50 Hydrogen cylinder
60 Nitrogen cylinder
100 Formate production system
L1, L2, L3, L4, L5, L6, L7 Flow path

**Claims**

1.  A formate production method, comprising:

    a first step of producing a formate by causing a reaction between carbon dioxide and hydrogen in a solution containing a solvent, a catalyst dissolved in the solvent, and a metal salt or an organic salt; and
    a second step of separating, by a separation membrane, the catalyst from a reaction solution obtained in the first step, wherein
    the catalyst contains at least one metal element selected from the group consisting of metal elements belonging to Group 8, Group 9, and Group 10 of a periodic table.

2.  The formate production method according to claim 1, wherein a base concentration in the reaction solution in the second step is 0.1 to 0.8 mol/L.

3.  The formate production method according to claim 1 or 2, wherein a pH of the reaction solution in the second step is 6.3 to 7.3.

4.  The formate production method according to any one of claims 1 to 3, wherein a formate concentration with respect to a base concentration in the reaction solution in the second step is 0.5 or more.

**5.** The formate production method according to any one of claims 1 to 4, wherein the catalyst separated in the second step is reused in the first step.

**6.** The formate production method according to any one of claims 1 to 5, wherein the separation membrane includes a separation membrane unit including a reverse osmosis membrane.

**7.** The formate production method according to any one of claims 1 to 6, wherein the metal element is Ru, Ir, Fe, Ni, or Co.

**8.** The formate production method according to any one of claims 1 to 7, wherein the metal element is Ir or Ru.

**9.** A formate production system that produces a formate, the system comprising:

a formate production device configured to produce a formate by causing a reaction between carbon dioxide and hydrogen in a solution containing a solvent, a catalyst dissolved in the solvent, and a metal salt or an organic salt; and
a separation device configured to separate the catalyst from a reaction solution obtained by the reaction, wherein the catalyst contains at least one metal element selected from the group consisting of metal elements belonging to Group 8, Group 9, and Group 10 of a periodic table.

**FIG. 1**

# FIG. 2

<table>
<tr><td colspan="2">**INTERNATIONAL SEARCH REPORT**</td><td>International application No.</td></tr>
<tr><td colspan="2"></td><td>PCT/JP2021/007388</td></tr>
</table>

| A. CLASSIFICATION OF SUBJECT MATTER |
|---|
| Int.Cl. C07C51/00(2006.01)i, B01D61/02(2006.01)i, B01D61/14(2006.01)i, B01J31/24(2006.01)i, B01J31/40(2006.01)i, B01J38/00(2006.01)i, C07C53/06(2006.01)i |
| FI: C07C51/00, B01D61/02500, B01D61/14500, B01J38/00301R, B01J31/24M, B01J31/40M, C07C53/06 |
| According to International Patent Classification (IPC) or to both national classification and IPC |

| B. FIELDS SEARCHED |
|---|
| Minimum documentation searched (classification system followed by classification symbols) |
| Int.Cl. C07C51/00, B01D61/02, B01D61/14, B01J31/24, B01J31/40, B01J38/00, C07C53/06 |

| Documentation searched other than minimum documentation to the extent that such documents are included in the fields searched |  |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

| Electronic data base consulted during the international search (name of data base and, where practicable, search terms used) |
|---|
| JSTPlus/JMEDPlus/JST7580(JDreamIII), JSTChina(JDreamIII) |

**C. DOCUMENTS CONSIDERED TO BE RELEVANT**

| Category* | Citation of document, with indication, where appropriate, of the relevant passages | Relevant to claim No. |
|---|---|---|
| Y | JP 2007-55915 A (NATIONAL INSTITUTE OF ADVANCED INDUSTRIAL SCIENCE & TECHNOLOGY) 08 March 2007 (2007-03-08), claims, examples, etc. | 1-9 |
| Y | JP 2004-217632 A (NATIONAL INSTITUTE OF ADVANCED INDUSTRIAL SCIENCE & TECHNOLOGY) 05 August 2004 (2004-08-05), claims, examples, particularly, example 5, etc. | 1-9 |
| Y | HIMEDA, Yuichiro et al., Interconversion between formic acid and H2/CO2 using rhodium and ruthenium catalysts for CO2 fixation and H2 storage, ChemSusChem, 2011, 4(4), 487-493, "Syntheses and properties", "Hydrogenation of CO2/bicarbonate under basic conditions", etc. | 1-9 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 April 2021 | 11 May 2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2021/007388

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2015-502922 A (BASF SE) 29 January 2015 (2015-01-29), claims, examples, etc. | 1-9 |
| Y | JP 2016-525925 A (EVONIK DEGUSSA GMBH) 01 September 2016 (2016-09-01), claims, examples, drawings, etc. | 1-9 |
| Y | JP 2016-193421 A (EVONIK DEGUSSA GMBH) 17 November 2016 (2016-11-17), claims, examples, drawings, etc. | 1-9 |
| Y | JP 2016-528223 A (EVONIK DEGUSSA GMBH) 15 September 2016 (2016-09-15), claims, drawings, etc. | 1-9 |

Form PCT/ISA/210 (second sheet) (January 2015)

| INTERNATIONAL SEARCH REPORT | International application No. |
|---|---|
| Information on patent family members | PCT/JP2021/007388 |

```
JP 2007-55915 A    08 March 2007       (Family: none)

JP 2004-217632 A   05 August 2004      (Family: none)

JP 2015-502922 A   29 January 2015     WO 2013/068389 A1
                                       CN 103917551 A
                                       KR 10-2014-0090183 A

JP 2016-525925 A   01 September 2016   JP 2018-61958 A
                                       US 2016/0082393 A1
                                       claims, examples, drawings
                                       WO 2014/183952 A1
                                       DE 102013208759 A1
                                       KR 10-2016-0007637 A
                                       CN 105377425 A

JP 2016-193421 A   17 November 2016    US 2016/0236150 A1
                                       claims, examples, drawings
                                       EP 3059005 A1
                                       KR 10-2016-0101881 A
                                       CN 105938518 A

JP 2016-528223 A   15 September 2016   US 2016/0158703 A1
                                       claims, drawings
                                       WO 2015/014741 A1
                                       EP 3027298 A1
                                       KR 10-2016-0036616 A
                                       CN 105579118 A
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016525925 T
- JP 2018114495 A

- JP 2020046665 A

**Non-patent literature cited in the description**

- **YUICHIRO HIMEDA ; NOBUKO ONOZAWA-KOMATSUZAKI ; SATORU MIYAZAWA ; HIDEKI SUGIHARA ; TAKUJI HIROSE ; KAZUYUKI KASUGA.** *Chem. Eur. J.,* 2008, vol. 14, 11076-11081

- **WAN-HUI WANG ; JONATHAN F. HULL ; JAMES T. MUCKERMAN ; ETSUKO FUJITA ; YUICHIRO HIMEDA.** *Energy Environ. Sci.,* 2012, vol. 5, 7923-7926
- **YUICHIRO HIMEDA ; NOBUKO ONOZAWA-KOMATSUZAKI ; HIDEKI SUGIHARA ; KAZUYUKI KASUGA.** *Organometallics,* 2007, vol. 26 (3), 702-712